# DEMANDE DE BREVET EUROPEEN

(11) **EP 2 112 138 A1**
(43) Date de publication de la demande: **28.10.2009**
(21) Numéro de dépôt: 09290285.7
(22) Date de dépôt: 17.04.2009
(51) Int. Cl.: C07D 215/32

(54) **Sels d'onium et leur utilisation pour la detection et le dosage des metaux**

(30) Priorité: 21.04.2008 FR 0802209
(71) Demandeur: Commissariat à l'Energie Atomique, Bâtiment D "Le Ponant" 25 rue Leblanc 75015 Paris (FR)
(72) Inventeur: Marchand, Giles, 38119 Pierre Chatel (FR); Loe Mie, Faidjiba, 38000 Grenoble (FR); Pucheault, Mathieu, 35220 Chateaubourg (FR); Vaultier, Michel, 35410 Chateaugiron (FR); Blanchard-Desce, Mireille, 35000 Rennes (FR)
(74) Mandataire: Corizzi, Valérie

(57) **Abrégé**

Sels d'onium à tâche spécifique de formule (I), procédé pour leur préparation et leur utilisation pour détecter et doser les métaux, en particulier les métaux lourds, dans un milieu aqueux.

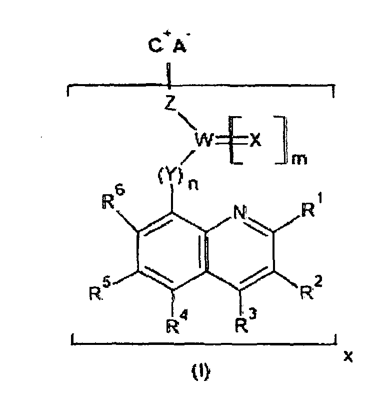

## Description

L'invention a pour objet de nouvelles molécules du type sels d'onium à tâche spécifique, un procédé pour leur préparation et leur utilisation pour détecter et doser les métaux, en particulier les métaux lourds, dans un milieu aqueux.

Le dosage des métaux lourds tels que par exemple Hg, Pb, Cd, dans l'eau est une nécessité du point de vue sanitaire et environnemental. En effet, sur les 41 éléments répondant à cette définition, 21 éléments sont toxiques pour l'homme et pour l'environnement.

Selon les procédés couramment employés, le dosage de métaux lourds dans une phase aqueuse peut être fait suivant deux méthodes :
Détection directe dans la phase aqueuse : Cette méthode de dosage fait appel à des techniques telles que l'ICP/MS (spectrométrie de masse à torche à plasma) ou l'AAS (spectroscopie d'absorption atomique). Ces techniques, bien que très sensibles, sont lourdes et difficilement intégrables dans un système portable. L'analyse des éléments se fait donc de façon déportée et non sur le terrain. Les systèmes portables connus font appel à une détection électrochimique (polarographie, CV (voltampérométrie cyclique), ASV (voltampérométrie à redissolution cathodique)), comme par exemple l'appareil Trace Detect ® commercialisé par la société Fondis Electronic. Ces techniques sont limitées par le fait que certains métaux n'ont pas de signature électrochimique dans la fenêtre électrochimique de l'eau (Al, Ti, Li...) ou ne sont pas électrodéposables sur une électrode (ASV). De plus, la sélectivité de la méthode de mesure (ici c'est la méthode de mesure qui apporte la sélectivité) s'avère parfois faible dans des milieux contenants plusieurs métaux ayant des réponses électrochimiques proches. Enfin, la fiabilité de telles mesures est limitée notamment en terme de reproductibilité pour des composés à de faibles concentrations. Une autre limitation de l'ASV réside dans le fait que cette technique est trop lente pour détecter des événements transitoires de pollution extrêmement rapide (il faut accumuler sur une électrode pour redissoudre) alors qu'une mesure optique permet une mesure en temps réel. Il existe également des détecteurs de plomb portables utilisant comme technique de détection les rayons X. La source de rayons X excite les électrons des couches profondes (K ou L) des métaux lourds. Ceux-ci relaxent cette excitation en émettant un rayonnement X à des énergies caractéristiques du métal. Les mesures sont rapides et la sensibilité est bonne, compatible avec les limites maximales admissibles en vigueur. Cependant, un problème lié à ce type de détecteur réside dans la dangerosité de la manipulation de ces tubes émetteurs de rayons X.
Quelle que soit la méthode employée, le dosage direct de métaux en milieu aqueux en dehors d'un laboratoire, et notamment en milieu naturel, s'avère souvent très problématique en raison de la présence de ces éléments sous forme de traces et des limitations des techniques actuelles en terme de sensibilité. En effet, les équipements de mesure en ligne connus atteignent un seuil de détection de quelques microgrammes par litre alors que les objectifs de la directive 2006/11/CE sont de l'ordre de quelques millièmes de microgrammes.

**Tableau 1 : évaluation de l'état chimique des eaux : valeurs seuils (µg/L)**

| | PNEC* pour les organismes d'eau douce (INERIS, 2007). | Valeurs seuils provisoires du bon état chimique (directive 2005/14/CE) | Valeurs seuils** délimitant des eaux de très bonne qualité (SEQ***-eau) |
|---|---|---|---|
| Cd | 0,21 | 5 | 0,004 |
| Cu | 1,6 | - | 0,1 |
| Hg | 0,24 | 1 | 0,007 |
| Pb | 5 | bf + 0,4 | 0,52 |
| Zn | 8,6 | - | 0,43 |

| | | | |
|---|---|---|---|
| *concentrations prévisibles sans effet. **pour une eau brute de dureté moyenne. *** SEQ : Système d'Evaluation de la Qualité des cours d'eaux bf : bruit de fond du dosage. | | | |

Ainsi, pour améliorer la précision et la sensibilité et/ou le seuil de détection de leur dosage, une phase d'extraction dans de faibles volumes de solvants organiques, afin de purifier partiellement et de concentrer les composés à doser, s'avère nécessaire.

Détection après concentration par extraction liquide/liquide : L'extraction liquide/liquide de métaux à partir d'une phase aqueuse par une phase organique permet de réaliser une concentration des métaux et d'atteindre ou d'abaisser pour des échantillons peu concentrés, le seuil de détection de la méthode de détection choisie. L'extraction efficace des métaux, du milieu aqueux vers le milieu organique peut-être améliorée par l'utilisation de molécules chélatantes, en solution dans le solvant organique. Le complexe résultant a davantage d'affinité avec la phase réceptrice que l'ion métallique seul. Cette extraction peut être sélective d'un métal donné, ou non sélective. Après extraction, le dosage se fait par les techniques précédemment citées (ICP/MS, AAS, électrochimie) avec les mêmes problèmes d'encombrement. Parfois encore, le complexant est fluorescent et la complexation du métal entraîne une modification de la longueur d'onde d'émission du complexant (Tetrahedron Letters Volume 48, Issue 37, 10 September 2007, pages 6527-6530) mais les procédés connus faisant appel à cette méthode présentent plusieurs inconvénients : la limite de détection est gênée par le phénomène d'offset (superposition partielle du pic produit par le complexant seul et du pic produit par le complexant lié au métal), des jeux de filtres sont nécessaires. Quel que soit le chélatant employé, le complexe ionique (chélatant +ion) doit être très peu soluble dans l'eau sinon il reste partiellement solubilisé en phase aqueuse, l'extraction est peu efficace et le dosage est biaisé.

Avec les solvants classiques, dans la plupart des cas, les rendements d'extraction sont faibles, car l'extraction est thermodynamiquement défavorisée (S. Dai et al., J. Chem. Soc. Dalton Trans., 1999, 1201-1202). De plus, la volatilité de ces solvants (SOV, solvants organiques volatils) rend difficile leur utilisation en très faible volume sans utilisation d'un capotage (comme par exemple un canal) ce qui complexifie le dispositif (Tokeshi M. et al., Anal. Chem. 2002, 1565-1571) et rend plus difficile la mise en oeuvre des procédés. En particulier, le traitement d'un grand nombre d'échantillons en parallèle s'avère compliqué. Afin de pallier à ces problèmes, il a été proposé l'utilisation de liquides ioniques (WO2004/005222). En effet, les liquides ioniques sont dotés de propriétés remarquables : *i*/ ce sont des solvants non volatils (leur tension de vapeur est négligeable) et ils peuvent donc être utilisés en volume très réduit ; *ii*/ ils peuvent être conçus pour être non miscibles avec l'eau en fonction de l'anion et ou du cation utilisé(s) et ils sont capables de solubiliser la plupart des molécules organiques et inorganiques ; *iii*/ leurs coefficients de partage (coefficient de distribution de Nernst) sont souvent élevés. Ainsi, des tests d'extraction d'ions Cs⁺ dans ces solvants contenant des chélatants comme des éthers couronnes (Luo H. et al., Anal. Chem. 2004, 3078-3083) ont montré une augmentation de l'efficacité d'extraction par rapport aux solvants habituellement utilisés (chloroforme). Le document DE3607982 décrit l'extraction de cations et d'anions à l'aide de sels d'onium. Cependant, les composés décrits dans ce document ne permettent pas de faire un dosage direct des ions détectés.

Le document Kumano et al., Int. J. Modern Physics, 20 (25, 26, 27), 2006, 4051-4056 décrit l'utilisation de 8-hydroxyquinoléine comme agent chélatant pour extraire des métaux. Mais ce procédé ne permet pas de faire une détection directe des espèces détectées.

Cependant, l'extraction par les liquides ioniques en présence d'une molécule chélatante présente également des inconvénients : *i*/ une fois le complexe ionique (chélatant + ion) réalisé, celui-ci peut éventuellement repasser en phase aqueuse car l'ion reste souvent hydraté et l'extraction est alors moins efficace ; *ii*/ la détection et le dosage du métal se font par les techniques précédemment citées avec les mêmes inconvénients. Si le 1^{er} de ces problèmes a été résolu en supportant les fonctions chélatantes sur des liquides ioniques afin de former des liquides ioniques à tâche spécifique (TSIL en anglais pour task specific ionic liquid) (Visser et al, chem. Commun., 2001, 135-136 ; Environ. Sci. Technol., 2002, 36, 2523-2529), le second problème subsiste. Dans cette dernière publication, la détection est faite par mesure de la radioactivité (²⁰³HgCl₂ et ¹⁰⁹CdCl₂), système encore plus lourd que ceux cités précédemment.

L'invention a pour avantage de résoudre l'ensemble des ces problèmes en proposant de nouvelles molécules qui permettent de réaliser à la fois une extraction très efficace des métaux à partir d'un milieu aqueux, et d'en effectuer une détection et éventuellement un dosage très sensible en faisant appel à des techniques non dangereuses pour le manipulateur et pour ceux qui l'entourent. Ces molécules, du type sel d'onium à tâche spécifique hydrophobe, comportant un groupement de la famille des quinoléines, permettent à la fois de capter les métaux dans un milieu aqueux, de les en extraire et d'émettre un signal de fluorescence conséquence de cette capture.

Un premier objet de l'invention est constitué des molécules de formule (I) ci-dessous : 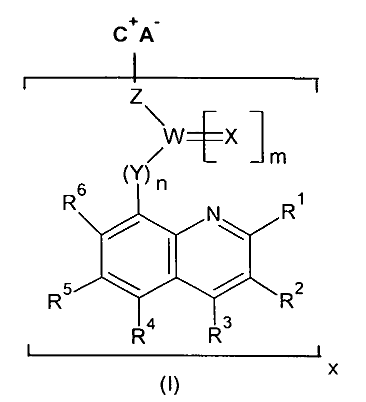 dans laquelle

X représente un groupement choisi parmi : O, NR, S, PR, Se ; et R représente un groupement choisi parmi : H, un groupement alkyle en C₁-C₁₂ comportant éventuellement un ou plusieurs hétéroatomes, un groupement alcényle en C₂-C₁₂ comportant éventuellement un ou plusieurs hétéroatomes, un groupement aryle en C₆-C₁₂ comportant éventuellement un ou plusieurs hétéroatomes, un groupement aralkyle en C₆-C₁₂ comportant éventuellement un ou plusieurs hétéroatomes ;

Y représente un groupement choisi parmi : O, S, NR', PR', CR^{'}R^{"}, un groupement alcane di-yle en C₁-C₁₂ comportant éventuellement un ou plusieurs hétéroatomes, un groupement alcène di-yle en C₂-C₁₂ comportant éventuellement un ou plusieurs hétéroatomes, un groupement aryle en C₆-C₁₂ comportant éventuellement un ou plusieurs hétéroatomes, un groupement aralcane di-yle en C₆-C₁₂ comportant éventuellement un ou plusieurs hétéroatomes ; et R', R^{"}, identiques ou différents, représentent un groupement choisi parmi : H, un groupement alkyle en C₁-C₁₂ comportant éventuellement un ou plusieurs hétéroatomes, un groupement alcényle en C₂-C₁₂ comportant éventuellement un ou plusieurs hétéroatomes,un groupement aralkyle en C₆-C₁₂ comportant éventuellement un ou plusieurs hétéroatomes, un groupement aryle en C₆-C₁₂ comportant éventuellement un ou plusieurs hétéroatomes ;
n est un entier choisi parmi : 0,1 ;
m est un entier choisi parmi : 1, 2 ;
x est un entier choisi parmi : 1 et 2 ;
W représente un atome choisi parmi : C et S;
Z représente un groupement choisi parmi : un groupement alcane di-yle en C₁-C₁₂ comportant éventuellement un ou plusieurs hétéroéléments, un groupement alcène di-yle en C₂-C₁₂ comportant éventuellement un ou plusieurs hétéroéléments, un groupement aralcane di-yle en C₆-C₂₀ comportant éventuellement un ou plusieurs hétéroéléments, un groupement aryle en C₆-C₁₂ comportant éventuellement un ou plusieurs hétéroéléments, le ou les hétéroéléments étant choisis parmi : Cl, Br, O, S, NR''', PR''' ; et R"' représente un groupement choisi parmi : H, un groupement alkyle en C₁-C₁₂, un groupement alcényle en C₂-C₁₂, un groupement aryle en C₆-C₁₂, un groupement aralkyle en C₆-C₁₂ ;

R¹, R², R³, R⁴, R⁵, R⁶, identiques ou différents, représentent chacun un groupement choisi parmi la liste suivante : H, Cl, Br, un groupement alkyle en C₁-C₁₂ comportant éventuellement un ou plusieurs hétéroéléments, un groupement alcényle en C₂-C₁₂ comportant éventuellement un ou plusieurs hétéroéléments, un groupement aryle en C₆-C₁₂ comportant éventuellement un ou plusieurs hétéroéléments, un groupement aralkyle en C₆-C₁₂ comportant éventuellement un ou plusieurs hétéroéléments, OH, un groupement OR⁷, NH₂, un groupement NR⁷R⁸, SH, un groupement SR⁷; et R⁷, R⁸ sont choisis indépendamment l'un de l'autre parmi la liste suivante : un groupement alkyle en C₁-C₁₂, un groupement alcényle en C₂-C₁₂, un groupement aryle en C₆-C₁₂, un groupement aralkyle en C₆-C_{12 ;} le ou les hétéroéléments étant choisis parmi : Cl, Br, O, S, NR"', PR"' ; et R"' représente un groupement choisi parmi : H, un groupement alkyle en C₁-C₁₂, un groupement alcényle en C₂-C₁₂, un groupement aryle en C₆-C₁₂, un groupement aralkyle en C₆-C₁₂.
→ **C⁺A⁻** représente un sel d'onium à tâche spécifique.

Lorsque x=1, **C⁺A⁻** peut être lié à Z soit par l'anion A⁻ soit par le cation C⁺.

Lorsque x=2, l'anion **A⁻** et le cation **C⁺** sont liés chacun à un groupement Z, les deux groupements 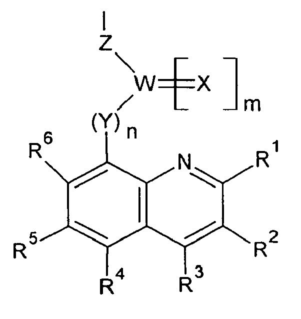 étant identiques ou différents.

L'expression « sel d'onium » désigne les sels d'ammonium de phosphonium, de sulfonium ainsi que tous les sels résultant de la quaternarisation d'une amine, d'une phosphine, d'un thioéther ou d'un hétérocycle contenant un ou plusieurs héteroatomes tels que S, N, P. Par « sel d'onium à tâche spécifique », on entend un sel d'onium dont soit le cation, soit l'anion est porteur d'une fonction capable de complexer un métal (suivant le cas on les désigne du nom de « cation fonctionnel » et/ou d'« anion fonctionnel »). L'anion et le cation peuvent être simultanément fonctionnels, en étant chacun lié à un groupement Z identique ou différent.

**C⁺** représente un cation fonctionnel ou non. **C**⁺ est préférentiellement choisi parmi les ions ammonium mais il peut également être choisi par exemple parmi : un pyrrolidinium, un phosphonium, un imidazolium, un pyridinium, un pipéridinium, un guanidinium.

**A-** représente un anion fonctionnel ou non. **A⁻** peut par exemple être choisi parmi les ions suivants : hexafluorophosphate (PF₆⁻), bis-(trifluorométhane sulfonamidure) (NTf₂⁻), un ion tris-(perfluoroalkyl) trifluorophosphate (FAP) tel que le tris (pentafluoroéthyl)trifluorophosphate (FAP), triflate (TfO⁻), nonaflate (NfO⁻), ou tout autre anion hydrophobe, l'ion tétrafluoroborate BF₄⁻, l'ion chlorure Cl⁻.

Selon une variante préférée de l'invention, les variables **C**⁺, **A⁻**, X, Y, W, R¹, R², R³, R⁴, R⁵, R⁶, sont choisies de telle sorte que le composé de formule (I) soit hydrophobe. On choisit avantageusement **C**⁺ et **A⁻** de façon à ce que le composé de formule (I) ne soit pas soluble dans l'eau. Ainsi, par exemple, si le cation est fonctionnel, l'anion est choisi de préférence parmi les anions hydrophobes, comme par exemple un ion hexafluorophosphate (PF₆⁻), un ion bis-(trifluorométhane sulfonamidure) (NTf₂⁻).

De façon préférée dans la formule (I), l'une ou plusieurs des conditions suivantes sont vérifiées :
X représente un groupement choisi parmi : O, S ; encore plus préférentiellement X représente O,
Y représente un groupement choisi parmi : O, S, NH, un groupement alcane di-yle en C₁-C₆ ; encore plus préférentiellement Y représente O, n = 1, m=1, x=1,
W représente un atome de carbone : C,
Z représente un groupement choisi parmi : un groupement alcane di-yle en C₁-C₆ comportant éventuellement un ou plusieurs hétéroéléments, aralcane di-yle en C₆-C₂₀ comportant éventuellement un ou plusieurs hétéroéléments, le ou les hétéroéléments étant choisis parmi : O, S, NH ; de préférence Z est choisi parmi les groupements alcane di-yles en C₁-C₆, comportant éventuellement un ou plusieurs atome d'oxygène dans ou à l'extrémité de la chaîne alkyle, les groupements aralcane di-yle en C₆-C₂₀, comportant éventuellement un ou plusieurs atome d'oxygène,
R¹, R², R³, R⁴, R⁵, R⁶, identiques ou différents, représentent chacun un groupement choisi parmi la liste suivante : H, un groupement alkyle en C₁-C₆, encore plus préférentiellement parmi H et un alkyle en C₁-C₃, et avantageusement parmi H et CH₃,
C⁺ représente un groupement 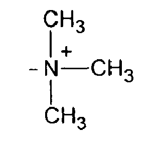 A⁻ représente un groupement choisi parmi : PF₆⁻ (hexafluorophosphate), NTf₂⁻ (bis-(trifluorométhane sulfonamidure), le tris-(pentafluoroéthyl)trifluorophosphate ; avantageusement A⁻ représente NTf₂⁻.

Les molécules suivantes constituent plus particulièrement un objet préféré de l'invention :

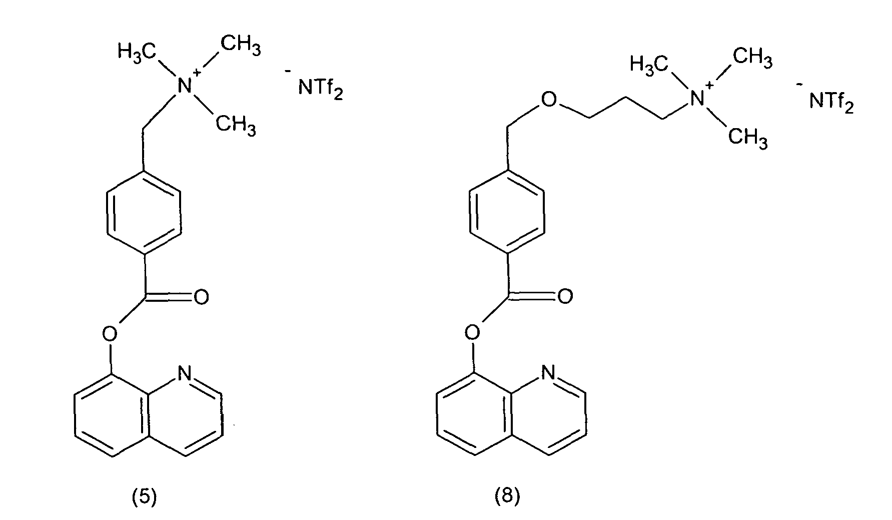

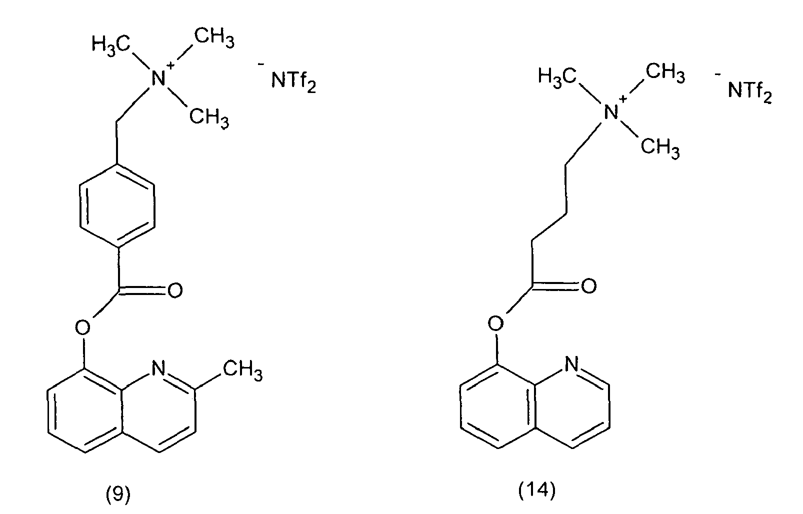

Le sel d'onium à tâche spécifique **(I)** présente les avantages suivants :
La fonction quinoléine substituée, capable de complexer le métal, étant portée par un sel d'onium hydrophobe, les risques de diffusion du complexe **(I)**-ion métallique dans la phase aqueuse sont très limités,
Le groupement quinoléine réalise à la fois la chélation et la détection par émission de fluorescence, permettant ainsi une détection directe du métal, par un dispositif de fluorescence approprié.

C'est un système qui fonctionne de la façon suivante : La quinoléine ne fluoresce pas ou peu en l'absence de métaux lourds dans le milieu, puis lorsqu'il y a complexation entre le métal et (I), il y a alors émission de fluorescence.

La synthèse des composés de formule (I) est faite à l'aide de méthodes de synthèse organique bien connues de l'homme du métier comme cela est illustré pour certains composés ci-dessous dans les exemples.

Les sels d'onium à tâche spécifique (I) peuvent être utilisés purs s'ils sont liquides ou éventuellement en solution dans un solvant, que ces sels soient sous forme solide, liquide, ou huileuse. Lorsqu'ils sont utilisés en solution, de préférence ils sont solubilisés dans un solvant hydrophobe ou peu miscible avec la phase aqueuse dans laquelle se trouve l'ion à extraire. Par exemple, ils peuvent être en solution dans un solvant halogéné tel que le chloroforme, le dichlorométhane, le dichloroéthane, CCl₄, un solvant fluoré, dans l'acétate d'éthyle, l'octanol, l'oxyde de mésityle. De façon avantageuse, les composés de formule (I) sont en solution dans une matrice liquide ionique à température ambiante (ou RTIL pour room temperature ionic liquid) hydrophobe.

L'expression « liquide ionique » désigne un sel ou un mélange de sels dont le point de fusion est compris entre -100°C et 250°C. Par matrice liquide ionique, on entend un liquide ionique, fonctionnalisé ou non, liquide à température ambiante, capable de solubiliser une ou plusieurs espèces chimiques telles que les sels minéraux ou organiques (tels que des sels d'onium à tâche spécifique), les molécules organiques, les polymères d'origine naturelle ou synthétique. Cette matrice liquide ionique peut être constituée d'un liquide ionique pur ou être un mélange de plusieurs liquides ioniques, fonctionnalisé(s) ou non, elle peut également être constituée d'un mélange d'un ou de plusieurs solvants hydrophobes tels qu'énumérés ci-dessus avec un ou plusieurs liquides ioniques. Parmi les liquides ioniques les plus connus, on peut citer les N,N-dialkylimmidazolium, les N-alkylpyridinium, les N,N-dialkylpyrrolidinium, les N,N-dialkylpipéridinium, les N,N trialkylammonium (Wasserscheid P. et al., Ionic liquids in synthesis, VCH : Weinheim, 2003 ; Wasserscheid P. et al., Chem. Rev 1999, 99, 2071 ; Rogers R.D., Seddon K.R. ACS Symposium Series 818, American Chemical Society, Washington DC, 2002 ; Rogers R.D., Seddon K.R. ACS Symposium Series 856, American Chemical Society, Washington DC, 2003 ; Bates E.D., J. Am. Chem. Soc. 2002, 124, 926). De façon avantageuse, on utilise un liquide ionique choisi parmi ceux qui ne possèdent pas de squelette aromatique et dont l'absorbance ne gêne pas ou peu l'excitation ou l'émission des molécules (I), comme par exemple les N,N-dialkylpyrrolidinium, les N,N-dialkylpipéridinium, les N,N trialkylammonium.

Un autre objet de l'invention est donc une composition comprenant au moins une molécule (I) telle que décrite ci-dessus et au moins un solvant. De telles compositions, tout comme le composé (I) pur, sont susceptibles d'être utilisées dans un procédé d'extraction, de détection et de dosage de métaux à partir d'une composition aqueuse.

Un autre objet de l'invention est un procédé d'extraction de métaux dans une composition aqueuse, ce procédé comportant au moins une étape de mise en contact de la composition aqueuse dans laquelle sont présents le ou les métaux, avec un composé de formule (I), ce composé de formule (I) pouvant être utilisé pour la mise en oeuvre de cette étape soit pur, lorsqu'il est liquide à la température ambiante, soit en solution. De préférence, cette mise en contact comporte au moins une étape de mélange : Par exemple, on peut placer les deux compositions dans un même récipient et agiter l'ensemble à l'aide d'un agitateur mécanique. On peut aussi utiliser un récipient clos dans lequel on place les deux compositions et on agite l'ensemble à l'aide de moyens d'agitation manuels ou mécaniques. On peut aussi prévoir d'utiliser un dispositif de circulation des fluides dans lequel on fait se rencontrer les flux de chacune des compositions de façon à permettre l'échange entre les deux phases. Notamment on peut utiliser des dispositifs tels que ceux qui ont été décrits dans WO2007/138180. Dans le cas de microsystèmes, le contact entre la molécule (I) et l'ion métallique peut-être réalisé par diffusion à l'interface entre le solvant hydrophobe et l'eau.

De façon avantageuse, le procédé de l'invention est mis en oeuvre au sein d'un dispositif microfluidique. De tels dispositifs présentent en effet l'avantage d'être aisément transportables et de n'utiliser que de petites quantités de produits (échantillon à tester et chélatant/détecteur). Ils sont donc aussi économiques et écologiques. Un tel dispositif comporte au moins une chambre de mélange dans laquelle l'échantillon à tester et le chélatant/détecteur de formule (I) (pur ou en composition) sont mis en contact. La chambre de mélange peut avoir n'importe quelle configuration, dès lors qu'elle permet la mise en contact des deux compositions, et notamment, il peut s'agir d'un canal de circulation des fluides. De tels dispositifs sont décrits notamment dans Tokeshi et al., Anal. Chem. 2002, 74, 1565-1571. Selon une autre variante préférée le procédé de l'invention est mis en oeuvre dans un tube à essai ou dans une plaque à puits.

Ce procédé peut être utilisé pour la capture et la détection de tous les composés métalliques, et notamment il permet la capture et la détection des métaux (éléments trace métalliques ou ETM) suivants : fer (Fe), plomb (Pb), mercure (Hg), uranium (U), chrome (Cr), cuivre (Cu), argent (Ag), or (Au), zinc (Zn), titane (Ti), nickel (Ni), cadmium (Cd), magnésium (Mg), calcium (Ca), mais aussi des autres métaux polluants tels que : aluminium (Al), gallium (Ga), germanium (Ge), arsenic (As), sélénium (Se), rubidium (Rb), strontium (Sr),yttrium (Y), zirconium (Zr), niobium (Nb), molybdène (Mo), technetium (Tc), ruthénium (Ru), rhodium (Rh), palladium (Pd), indium (In), étain (Sn), antimoine (Sb), tellure (Te), césium (Cs), barium (Ba), luthénium (Lu), hafnium (Hf), tantale (Ta), tungstène (W), rénium (Re), osmium (Os), iridium (Ir), platine (Pt), tallium (Tl), plutonium (Pu).

L'invention peut également être mise en oeuvre dans le cadre de la détection de métaux dans un liquide hydrophile. Dans ce cas, A⁻ sera choisi parmi BF₄⁻(tétrafluoroborate) ou parmi les ions halogénés (par exemple Cl⁻). Les groupements X, Y, W, R¹, R², R³, R⁴, R⁵, R⁶, sont choisis de telle sorte que le composé soit hydrophile.

Un autre objet de l'invention est un kit pour la détection de métaux en milieu aqueux comprenant un dispositif microfluidique et au moins un composé de formule (I), qu'il soit sous forme pure ou dans une composition.

La mise en contact de l'échantillon à tester, sous forme d'une composition aqueuse, et du chélatant/détecteur de formule (I) entraîne la chélation des métaux présents dans l'échantillon à tester par le chélatant, et leur extraction de la phase aqueuse. Cette mise en contact entraîne également l'émission d'un signal de fluorescence sous l'effet d'une source d'excitation, délivrant un signal lumineux dont la longueur d'onde dépend du métal, et est généralement comprise entre 280 et 380 nm. L'intensité de l'émission de fluorescence permet de quantifier, après étalonnage à l'aide d'un échantillon de référence, la concentration en métaux dans la phase aqueuse initiale.

Un autre objet de l'invention est donc un procédé de détection de métaux dans une composition aqueuse, ce procédé comportant au moins une étape de mise en contact de la composition aqueuse dans laquelle sont présents le ou les métaux, avec un composé de formule (I), et au moins une étape de mesure de fluorescence.

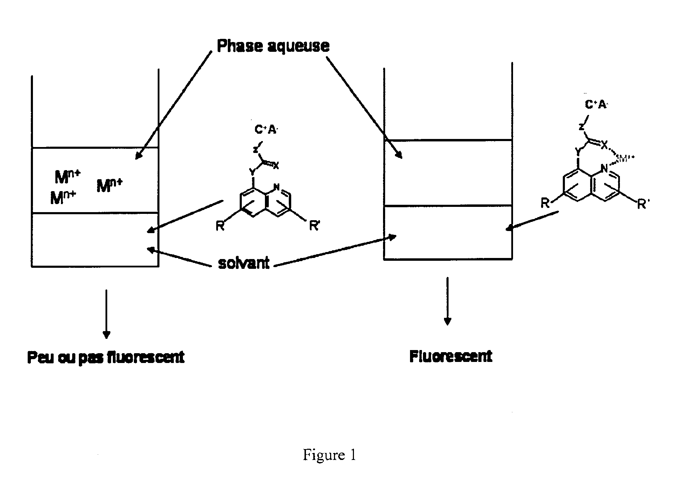

Un tel procédé est illustré schématiquement sur la figure 1 : A gauche, une phase aqueuse contenant des ions métalliques et une phase solvant contenant une molécule de formule (I) avant extraction liquide/liquide. La molécule (I) ne fluoresce pas ou peu. A droite, après extraction liquide/liquide et complexation d'ions métalliques par la molécule (I), la phase solvant devient fluorescente.

Un autre objet de l'invention est un kit pour la détection de métaux en milieu aqueux comprenant au moins un composé de formule (I), qu'il soit sous forme pure ou dans une composition, et un détecteur de fluorescence.

### EXEMPLES

### Figures :

Figure 1 : représentation schématique d'un procédé d'extraction et de détection,
Figure 2A : fluorescence de **(5)** en fonction de la quantité de Hg(ClO₄)₂,
Figure 2B : rapport des intensités de fluorescence entre le complexe M(ClO₄)₂ avec M variable et Hg(ClO₄)₂,
Figure 3A : fluorescence de **(8)** dans l'acétonitrile avec ajout de CuCl₂,
Figure 3B : rapport des intensités de fluorescence entre le complexe MCl₂ avec M variable et HgCl₂,
Figure 4 : fluorescence de **(8)** dans [BMP][NTf₂] avant extraction a), après extraction avec une solution aqueuse de HgCl₂ b), témoin avec de l'eau distillée c), λ_{exc} = 380 nm.
Figure 5 : fluorescence de **(5)** dans [BMP][NTf₂] après extraction selon la quantité de Hg(ClO₄)₂ contenue dans la phase aqueuse, λ_{exc} = 313 nm.
Figure 6A : Représentation schématique du circuit microfluidique
Figure 6B : Photographie du circuit microfluidique (agrandissement :)
Figure 7 : Fluorescence de **(5)** dans le [bmp][NTf₂](IL), après extraction de [Hg(ClO₄)₂] dissout dans l'eau, dans le microsystème. Dₑₐᵤ = 4 µL/min, D_{IL} = 0.02 µL/min.

### Matériel et méthodes :

Les solvants anhydres et les réactifs proviennent de Sigma-Aldrich et Roth. Ils ont été utilisés tels quels. Les spectres de RMN ¹H et ¹³C ont été enregistrés sur un spectromètre BRUCKER Avance 200 (200.13 MHz pour le proton, 50 MHz pour le carbone).

Les spectres d'émission de fluorescence sont obtenus sur un spectrofluorimètre PERKIN ELMEN LS50B

### 1- Synthèses

### Exemple n°1

Le composé (5) a été synthétisé conformément au schéma de synthèse exposé dans le schéma 1.

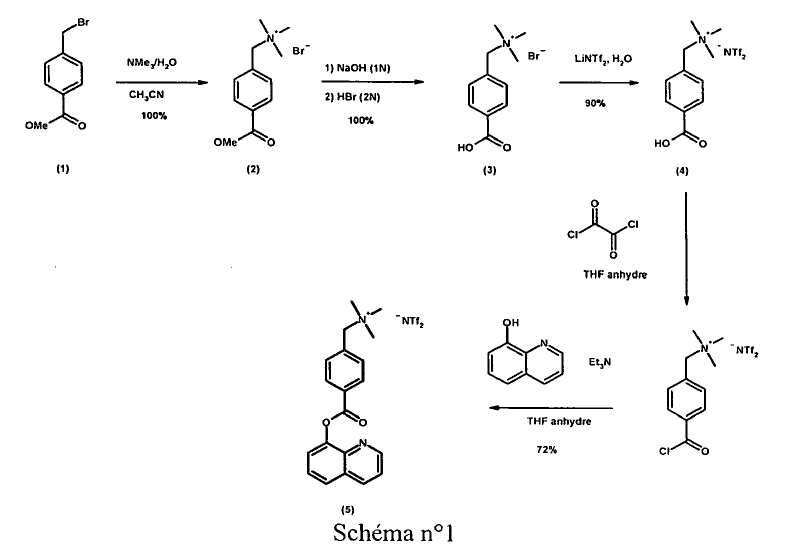

**Synthèse de (2).** Dans un Schlenk, une solution de triméthylamine (45% dans l'eau, 2 équivalents) est ajoutée en une fois à une solution de méthyl-4-(bromométhyl)-benzoate **(1)** (1 équivalent) dans l'acétonitrile (20 mL). Le mélange réactionnel est agité et chauffé à 70°C durant 15 heures. Le solvant et l'excès de triméthylamine sont ensuite évaporés à la pompe à palettes. Le résidu est lavé à l'éther (4 x 10mL) puis de nouveau séché à la pompe à palettes pour obtenir un solide blanc **2** (Rdt : 100%).

**Synthèse de (3).** 1 équivalent de **(2)** est mis en solution dans NaOH aqueux (1M, 1 équivalent). De l'eau déionisée est ajoutée jusqu'à dissolution complète de (2) Le mélange réactionnel est agité et chauffé à 80°C durant 15h. Après refroidissement jusqu'à la température ambiante, HBr (2N, 1 équivalent) est introduit. L'agitation est maintenue durant 15 min puis l'eau est évaporée à la pompe à palettes, en chauffant à 60°C, à l'aide d'un bain d'huile, durant 2 heures. Le résidu est lavé à l'éther (3 x 10mL) puis séché sous vide (pompe à palettes) durant 3h pour obtenir le solide blanc **(3)** (Rdt : 100%).

**Synthèse de (4).** Une solution de LiNTf₂ (2 équivalents) dans l'eau est ajoutée à **(3)** (1 équivalent). Un précipité blanc apparaît. Le mélange réactionnel est agité 3 heures, à température ambiante, puis filtré sur fritté (porosité 4). Le résidu obtenu est lavé (3 x 20 mL) à l'eau puis séché sous vide (pompe à palettes) en chauffant à 60°C à l'aide d'un bain d'huile durant 3h, pour obtenir un solide blanc **(4)** (Rdt : 76%).

**Synthèse de (5).** 6 gouttes de DMF anhydre sont ajoutées à une solution de **(4)** (1 équivalent) dans le THF anhydre (10mL). Après refroidissement à 0°C, le chlorure d'oxalyle (1.5 équivalent) est ajouté goutte à goutte. L'agitation est maintenue 15 min à 0°C, puis à température ambiante jusqu'à la fin du dégagement gazeux (3h). Le solvant et l'excès de chlorure d'oxalyle sont évaporés sous vide (pompe à palettes). Le résidu obtenu est de nouveau dissout dans le THF anhydre, la 8-hydroxyquinoléine puis la triéthylamine, fraîchement distillée, sont ajoutées. Le mélange réactionnel est agité 4h à température ambiante, puis filtré sur fritté (porosité 4). Le filtrat est concentré à l'évaporateur rotatif, puis dissout dans le dichlorométhane (20mL). La phase organique obtenue est lavée (3 x 10 mL) à l'eau déionisée, puis concentrée à l'évaporateur rotatif et lavé (3 x 10 mL) à l'éther pour obtenir un solide blanc verdâtre **(5)** (Rdt : 72%, Pf = 120°C). RMN ¹H (CD₃CN) δ 8.81 (dd, ³*J* = 4.1 Hz, ⁴*J* = 1.6 Hz, 1H), 8.38 (d, ³*J* = 8.3 Hz, 2H), 8.37 (dd, ³*J* = 8.3 Hz, ⁴*J* = 1.6 Hz, 1H), 7.92 (dd, ³*J* = 6.0 Hz, ³*J* = 3.5 Hz, 1H), 7.74 (d, ³*J* = 8.3 Hz, 2H), 7.66 (dd, ³*J* = 6.0 Hz, 1H), 7.65 (d, ³*J* = 3 .5 Hz, 1H), 7.52 (dd, ³*J* = 8.3 Hz, ³*J* = 4.1 Hz, 1H), 4.5 (s, 2H), 3.06 (s, 9H) ; RMN ¹³C (CD₃CN) δ 165.54, 151.53, 148.30, 141.67, 137.17, 134.40, 133.83, 132.44, 131.50, 130.41, 127.38, 127.35, 123.07, 122.46, 120.81 (q, *J*_{CF}=320.6 Hz), 69.4, 53.5 (t, *J*_{CN} = 3.9 Hz). MS (C⁺) 321.1.

### Exemple n°2

La synthèse du composé **(8)** est réalisée conformément au procédé décrit dans le schéma n°2

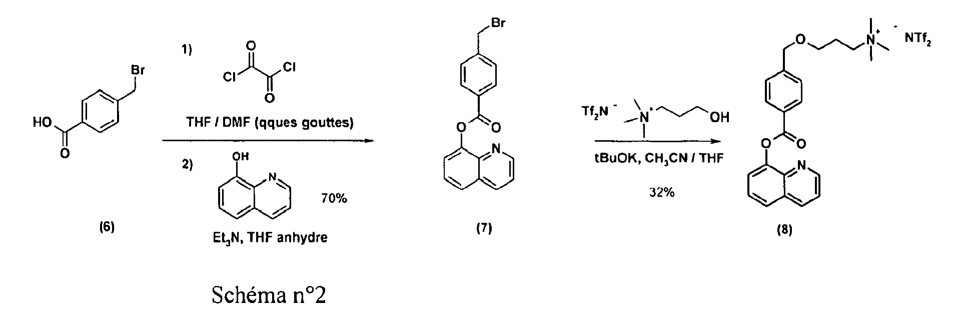

**Synthèse de (7).** 6 gouttes de DMF anhydre sont ajoutées à une solution d'acide 4-bromométhyl-benzoïque **(6)** (1 équivalent) dans le THF anhydre (10 mL). Après refroidissement à 0°C, le chlorure d'oxalyle (1.5 équivalent) est ajouté goutte à goutte. L'agitation est maintenue 15 min à 0°C, puis à température ambiante jusqu'à la fin du dégagement gazeux (3h). Le solvant et l'excès de chlorure d'oxalyle sont évaporés à la pompe à palette. Le résidu obtenu est de nouveau dissout dans le THF anhydre, la 8-hydroxyquinoléine puis la triéthylamine, fraîchement distillée, sont ajoutées. Le mélange réactionnel est agité 4h à température ambiante, puis filtré sur fritté (porosité 4). Le filtrat est concentré à l'évaporateur rotatif. **(7)** est isolé par purification sur colonne de silice (éluant CH₂Cl₂ / AcOEt 9 :1) pour obtenir un solide blanc nacré (Rdt : 70%). PF = 137°C

**Synthèse de (8).** Sous argon, la base tBuOK (1.1 équivalent) est ajoutée à une solution de bis-trifluorométhanesulfonamidure de (3-hydroxy-propyl)-triméthylammonium dans l'acétonitrile anhydre. Après 1h30 d'agitation à température ambiante, une solution de **(7)** dans un mélange THF / CH₃CN (1 :1) est ajouté au milieu réactionnel. L'agitation est maintenue 24h à température ambiante, puis le mélange réactionnel est filtré à l'aide d'un papier-filtre. Le filtrat est concentré puis lavé 5 fois (5 x 10 mL) à l'éther. **(9)** est isolé par purification sur colonne de silice C₁₈ (éluant CH₃CN/CH₃OH/H₂O 3 :3 :4) pour obtenir une huile visqueuse marron (Rdt : 32%). RMN ¹H (Acétone-d₆) δ 8.96 (dd, ³*J* = 4.1 Hz, ⁴*J* = 1.6 Hz, 1 H), 8.34 (dd, ³*J* = 8.3 Hz, ⁴*J* = 1.6 Hz, 1H), 8.11 (d, ³*J* = 8.3 Hz, 2H), 7.80 (d, ³*J* = 8.3 Hz, 2H), 7.60 (d, ³*J* = 4.1 Hz, 1H), 7.57-7.49 (m, 2H), 7.31 (dd, ³*J* = 6.7 Hz, ⁴*J* = 2.2 Hz, 1H), 5.52 (s, 2H), 4.53 (t, ³*J* = 5.7 Hz, 2H), 3.94-3.83 (m, 2H), 3.47 (s, 9H), 2,6-2.46 (m, 2H). RMN ¹³C (Acétone-d₆) δ 167.09, 156.10, 150.71, 144.95, 142.34, 137.29, 131.22, 131.163, 130.84, 128.26, 128.26, 123.43, 121.96, 121.7 (q, *J*_{CF} =321.4 Hz), 112.04, 71.24, 65.69 (t, *J*_{CN} = 3.5 Hz), 63.05, 54.51 (t, *J*_{CN} = 3.9 Hz), 24.32. MS (C⁺) 379.3

### Exemple n°3

La synthèse du composé **(9)** est réalisée conformément au procédé décrit dans le schéma n°3 :

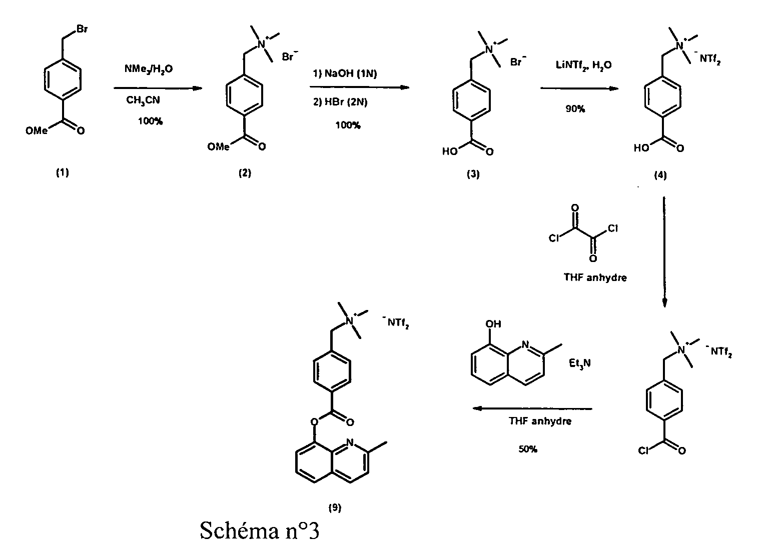

**Synthèse de (9)**. 6 gouttes de DMF anhydre sont ajoutées à une solution de **(4)** (1 équivalent, synthèse décrite précedemment) dans le THF anhydre (10mL). Après refroidissement à 0°C, le chlorure d'oxalyle (1.5 équivalent) est ajouté goutte à goutte. L'agitation est maintenue 15 min à 0°C, puis à température ambiante jusqu'à la fin du dégagement gazeux (3h). Le solvant et l'excès de chlorure d'oxalyle sont évaporés sous vide (pompe à palettes). Le résidu obtenu est de nouveau dissout dans le THF anhydre, la 8-hydroxyquinaldine puis la triéthylamine, fraîchement distillée, sont ajoutées. Le mélange réactionnel est agité 4h à température ambiante, puis filtré sur fritté (porosité 4). Le filtrat est concentré à l'évaporateur rotatif, lavé (3 x 10 mL) à l'éther puis dissout dans le dichlorométhane (20mL). La phase organique obtenue est lavée (3 x 10 mL) à l'eau déionisée, puis concentrée à l'évaporateur rotatif pour obtenir un solide blanc verdâtre **(9)** (Rdt : 50%, Pf : 120°C). RMN ¹H (Acétone-d₆) δ 8.45 (d, ³*J* = 8.3 Hz, 2H), 8.35 (d, ³*J* = 8.6 Hz, 1H), 8.00 (d, ³*J* = 8.3 Hz, 2H), 7.94 (dd, ³*J* = 5.4 Hz, ³*J* = 4.1 Hz, 1H), 7.66 (d, ³*J* = 5.4 Hz, 1H), 7.51 (d, ³*J* = 8.6 Hz, 1H), 5.01 (s, 2H), 3.52 (s, 9H), 2.63 (s, 3H). RMN ¹³C (Acétone-d₆) : δ 165.85, 160.9, 148.85, 137.8, 135.1, 134.8, 133.6, 132.25, 129.6, 127.6, 127.0, 124.4, 123.1 (q, *J*_{CF} =320.6 Hz), 70.2, 54.2 (t, *J*_{CN} = 3.9 Hz), 26.4.

### Exemple n°4

La synthèse du composé **(14)** est réalisée conformément au procédé décrit dans le schéma n°4

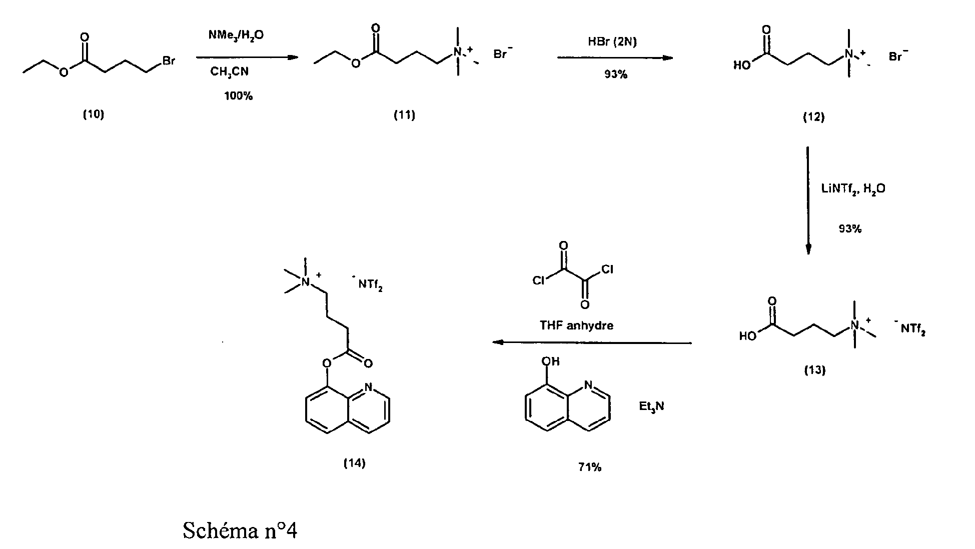

**Synthèse de (11).** Dans un Schlenk, une solution de triméthylamine (45% dans l'eau, 2 équivalents) est ajoutée en une fois à une solution d'éthyl-4-bromobutyrate (1 équivalent) dans l'acétonitrile (20 mL). Le mélange réactionnel est agité et chauffé à 80°C durant 15 heures. Le solvant et l'excès de triméthylamine sont ensuite évaporés à la pompe à palettes. Le résidu est lavé à l'éther (4 x 10mL) puis de nouveau séché à la pompe à palettes pour obtenir un solide blanc **(12)** (Rdt : 100%).

**Synthèse de (12).** 1 équivalent de **(11)** est mis en solution dans HBr aqueux (2N, 1 équivalent). De l'eau déionisée est ajoutée jusqu'à dissolution complète de **(11).** Le mélange réactionnel est agité et chauffé à 80°C durant 15h, puis l'eau est évaporée à la pompe à palettes, en chauffant à 60°C, à l'aide d'un bain d'huile, durant 2 heures. Le résidu est lavé à l'éther (3 x 15mL), puis une fois à l'acétone (15 mL) et séché sous vide (pompe à palettes) pour obtenir le solide blanc **(12)** (Rdt : 93%, Pf=196°C).

**Synthèse de (13).** Une solution de LiNTf₂ (1.5 équivalents) dans l'eau est ajoutée à **(12)** (1 équivalent). Le mélange réactionnel est agité 3 heures, à température ambiante. Deux phases sont obtenues et transférées dans une ampoule à décanter. La phase inférieure (liquide ionique) est séparée puis la phase aqueuse est lavée 2 fois (2x10 mL) au CH₂Cl₂. Après évaporation du solvant. Une huile légèrement jaune est obtenue **(13)** (Rdt : 71 %).

**Synthèse de (14).** 6 gouttes de DMF anhydre sont ajoutées à une solution de **(13)** (1équivalent) dans le THF anhydre (10mL). Après refroidissement à 0°C, le chlorure d'oxalyle (1.5 équivalent) est ajouté goutte à goutte. L'agitation est maintenue 15 min à 0°C, puis à température ambiante jusqu'à la fin du dégagement gazeux (2h). Le solvant et l'excès de chlorure d'oxalyle sont évaporés sous vide (pompe à palettes). Le résidu obtenu est de nouveau dissout dans le THF anhydre, la 8-hydroxyquinoléine puis la triéthylamine, fraîchement distillée, sont ajoutées. Le mélange réactionnel est agité 4h à température ambiante, puis filtré sur fritté (porosité 4). Le filtrat est concentré à l'évaporateur rotatif, lavé (3 x 10 mL) à l'éther puis dissout dans le dichlorométhane (20mL). La phase organique obtenue est lavée 3 (3 x 10 mL) à l'eau déionisée, puis concentrée à l'évaporateur rotatif pour obtenir une huile très visqueuse vert-marron **(14)** (Rdt : 78%). RMN ¹H (acétone d₆) δ 8.98 (dd, ³*J* = 4.1 Hz, ⁴*J* = 1.6 Hz, 1H), 8.47 (dd, ³*J* = 8.6 Hz, ⁴*J* = 1.6 Hz, 1H), 7.96 (dd, ³*J* = 8.3 Hz, ⁴*J* = 1.6 Hz, 1H), 7.73-7.55 (m, 3H), 3.97-3.88 (m, 2H), 3.5 (s, 9H), 3 (t, ³*J* =, 2H), 2.42-2.57 (m, 2H), RMN ¹³C (acétone d₆) : δ 171.98, 152.08, 149.12, 142.51, 137.90, 131.27, 127.97, 127.74, 123.68, 123.04, 121.73 (*J*_{CF} =321.6 Hz), 67.24 (t, *J*_{CN} = 3.1 Hz), 54.53 (t, *J*_{CN} = 4.1 Hz), 20.23.

### Exemple n°5

La synthèse du composé **(15)** est réalisée conformément au procédé décrit dans le schéma n°5 :

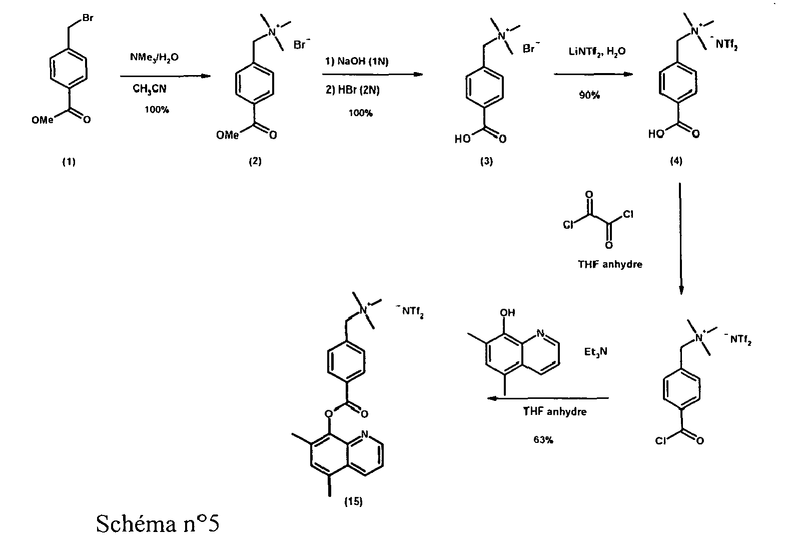

**Synthèse de (15).** 6 gouttes de DMF anhydre sont ajoutées à une solution de **(4)** (1 équivalent, synthèse décrite précédemment à l'exemple 1) dans le THF anhydre (10mL). Après refroidissement à 0°C, le chlorure d'oxalyle (1.5 équivalent) est ajouté goutte à goutte. L'agitation est maintenue 15 min à 0°C, puis à température ambiante jusqu'à la fin du dégagement gazeux (3h). Le solvant et l'excès de chlorure d'oxalyle sont évaporés sous vide (pompe à palettes). Le résidu obtenu est de nouveau dissout dans le THF anhydre, la 5,7-dimethyl-8-hydroxyquinoline puis la triéthylamine, fraîchement distillée, sont ajoutées. Le mélange réactionnel est agité 4h à température ambiante, puis filtré sur fritté (porosité 4). Le filtrat est concentré à l'évaporateur rotatif, lavé (3 x 10 mL) à l'éther puis dissout dans le dichlorométhane (20 mL). La phase organique obtenue est lavée (3 x 10 mL) à l'eau déionisée, puis concentrée à l'évaporateur rotatif pour obtenir une huile très visqueuse verdâtre **(15)** (Rdt : 63%). RMN ¹H (CD₃CN): δ 8.74 (d, ³*J* = 3.5 Hz, 1H), 8.36 (d, ³*J* = 7.6 Hz, 2H), 8.28 (d, ³*J* = 8.3 Hz, 1H), 7.68 (d, ³*J* = 7.6 Hz, 2H), 7.45 (dd, ³*J* = 8.3 Hz, ³*J* = 3.5 Hz, 1H), 7.35 (s, 1H), 4.46 (s, 2H), 3.04 (s, 9H), 2.64 (s, 3H), 2.38 (s, 3H). RMN ¹³C (CD₃CN ): δ 165.2, 150.8, 141.8, 134.4, 134.2, 133.9, 133.8, 132.5, 131.5, 131.3, 130.5, 127.8, 124.0, 121.7, 120.8 (q, *J*_{CF} =319.8 Hz), 69.4, 53.6 (t, *J*_{CN} = 3.9 Hz), 18.4, 16.3.

### Exemple n°6

La synthèse du composé \**(17)** est réalisée conformément au procédé décrit dans le schéma n°6 :

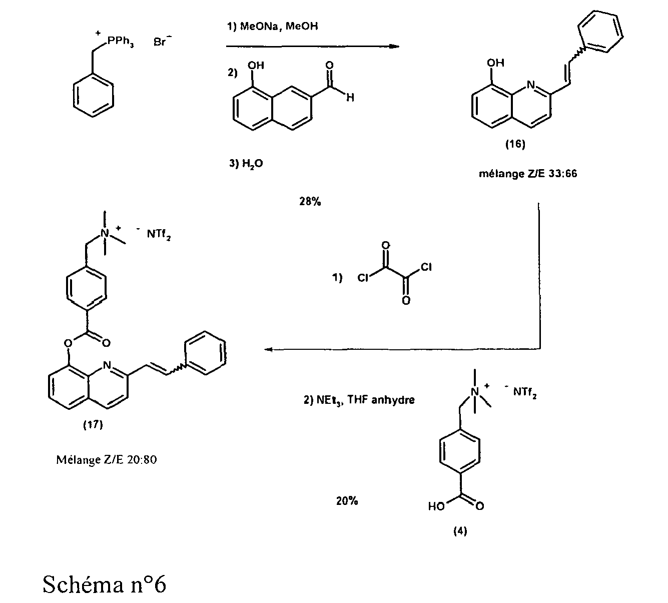

**Synthèse de (16), 2-styryl-8-hydroxyquinoline.** Le méthanolate de sodium (0.5 M dans le méthanol, 1.1 équivalent) est additionné lentement à une solution de bromure de benzyltriphenylphosphonium (1.1 équivalent) dans le méthanol (5 mL). Après 20 minutes d'agitation à température ambiante, la 2-carbaldéhyde-8-hydroxyquinoline est ajoutée. Le mélange est porté au reflux du méthanol durant 6h. Après refroidissement à température ambiante et hydrolyse par l'ajout de NaCl saturé (25mL), le milieu est neutralisé à l'aide d'HCl dilué. Les produits sont extraits par le dichlorométhane (3 x 30mL). La phase organique résultante est lavée à l'eau (2 x 20 mL) et séchée sur MgS04 puis concentrée à l'évaporateur rotatif. Un purification sur gel de silice (éluant : dichlorométhane) permet d'obtenir le composé **(16)** (mélange Z/E 33 :66) sous la forme d'une huile visqueuse jaune. (Rdt : 28%) RMN ¹H (CD₃CN) pour le Z : δ 8.25 (brs, 1H), 8.15 (d, ³*J* = 8.6 Hz, 1H), 7.49-7.34 (m, 8H), 7.1 ( dd, ³*J* = 6.7 Hz, ²*J* = 2.25 Hz, 1H), 7.09 (d, ¹*J* = 12.4 Hz, 1 H), 6.89 (d, ¹*J* = 12.4 Hz). Pour le E : 8.90 (brs, 1 H), 8.33 ( d, ³*J* = 8.6 Hz, 1H), 8.14 (d, ¹*J* = 16.2 Hz, 1H), 7.84 ( d, ³*J* = 8.6 Hz, 1H), 7-79-7.75 (m, 2H), 7.55 (d, ¹*J* = 16.2 Hz, 1H), 7.53-7.33 (m, 5H), 7.16 (dd, ³*J* = 6.7 Hz, ²*J* = 2.25 Hz, 1H).

**Synthèse de (17).** 6 gouttes de DMF anhydre sont ajoutées à une solution de **(4)** (1 équivalent, synthèse décrite précédemment à l'exemple 1) dans le THF anhydre (10mL). Après refroidissement à 0°C, le chlorure d'oxalyle (1.5 équivalent) est ajouté goutte à goutte. L'agitation est maintenue 15 min à 0°C, puis à température ambiante jusqu'à la fin du dégagement gazeux (3h). Le solvant et l'excès de chlorure d'oxalyle sont évaporés sous vide (pompe à palettes). Le résidu obtenu est de nouveau dissout dans le THF anhydre, la 2-styryl-8-hydroxyquinoline **(16)** (mélange Z/E 33 :66) puis la triéthylamine, fraîchement distillée, sont ajoutées. Le mélange réactionnel est agité 4h à température ambiante, puis filtré sur fritté (porosité 4). Le filtrat est concentré à l'évaporateur rotatif, lavé (3 x 10 mL) à l'éther puis dissout dans le dichlorométhane (20 mL). La phase organique obtenue est lavée (3 x 10 mL) à l'eau déionisée, puis concentrée à l'évaporateur rotatif pour obtenir une huile visqueuse verdâtre (17) (mélange Z/E 20:80, Rdt : 20%, Pf :). RMN ¹H (CD₃CN) pour le mélange: δ 8.45 (d, ³J = 8.3 Hz, 2H_{E}), 8.32 (d, ³J = 8.6 Hz, 1H_{E}), 8.26 (d, ³J = 8.3 Hz, 2H_{Z}), 8.13 (d, ³J = 8.6 Hz, 1H_{Z}), 7.87-7.18 (m, 13H_{E}+ 11H_{Z}), 6.87 (d, ¹*J* = 12.7 Hz, 1 H_{Z}), 6.67 (d, ¹*J* = 12.7 Hz, 1 H_{Z}), 4.54 (s, 2H_{E} ), 4.49 (s, 2H_{Z}), 3.08 (s, 9H_{E}), 3.06(s, 9H_{Z}).

### 2. Mesures spectrométriques

L'objectif de ces mesures est de montrer que la molécule de formule générale **(I)** permet à la fois la chélation et la détection directe par émission de fluorescence indépendamment de l'extraction.

### Exemple de détection sélective

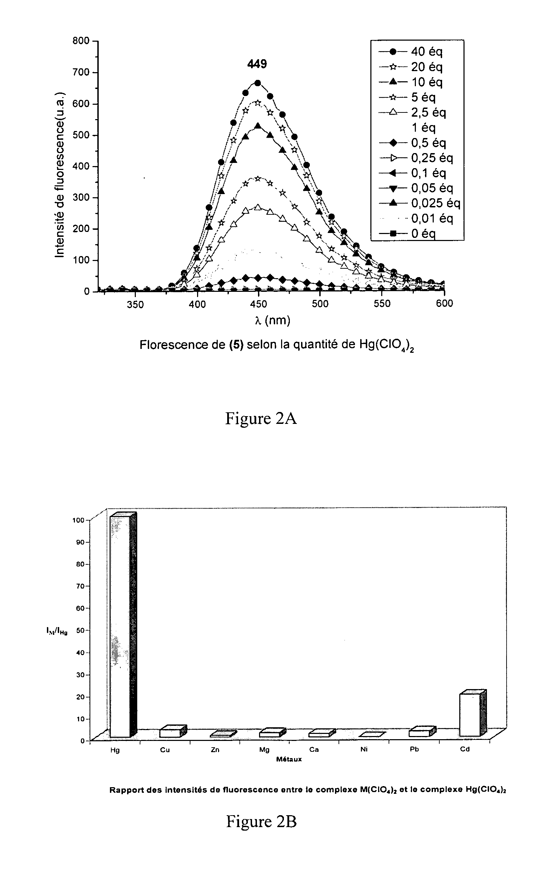

Une solution de **(5)** dans l'acétonitrile (c = 10⁻⁵M) est titrée en augmentant la quantité de sels métalliques dissous dans l'eau. Après chaque ajout de métal, les mesures de fluorescence sont réalisées entre 315 et 600 nm (λ_{exc} = 310 nm). Le nombre d'équivalents de sel métallique varie entre 0 et 40 équivalents de ligand **(5).** C'est-à-dire que le rapport des concentrations [sel métallique]/[ligand (5)] varie entre 0 et 40. Comme montré dans la figure 2, le signal de fluorescence à 449 nm augmente lorsque le nombre d'équivalents de Hg(ClO₄)₂ augmente. Les tests de sélectivité avec d'autres métaux ont montré que l'ordre de sélectivité est le suivant : Hg²⁺>>Cd²⁺>Cu²⁺>Pb²⁺>Mg²⁺>Ca²⁺>Zn²⁺>Ni²⁺.

### Exemple de détection non sélective

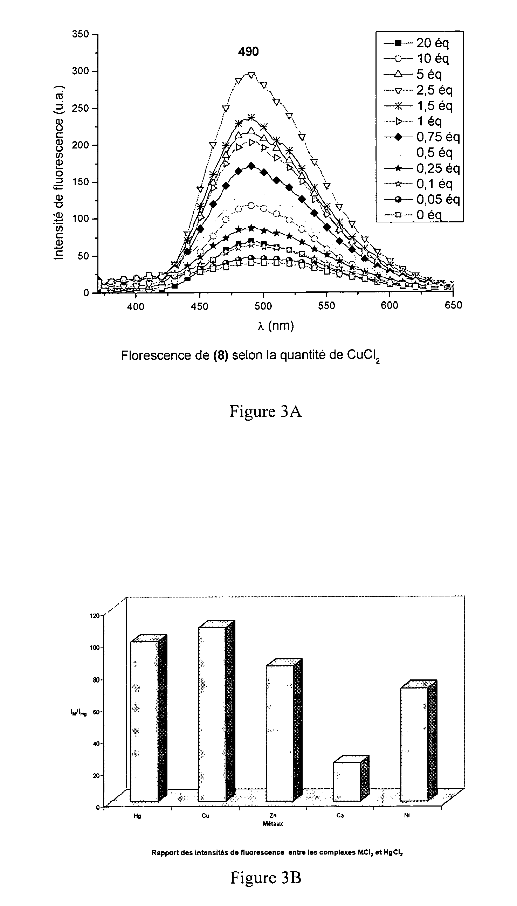

Une solution de **(8)** dans l'acétonitrile (c = 5.10⁻⁴M) est titrée en augmentant la quantité de sels métalliques (CuCl₂) dissous dans l'eau. Après chaque ajout de métal, les mesures de fluorescence sont réalisées entre 370 et 650 nm (λ_{exc} = 365 nm). Le nombre d'équivalents de sel métallique varie entre 0 et 40 équivalents de ligand **(8),** c'est-à-dire que le rapport des concentrations [sel métallique]/[ligand (5)] varie entre 0 et 40. Comme montré dans la figure 3, le signal de fluorescence à 490 nm augmente lorsque le nombre d'équivalents de CuCl₂ augmente. Les tests de sélectivité avec d'autres métaux ont montré que cette molécule **(8)** n'était pas sélective d'un métal en particulier.

### Exemple d'extraction phase aqueuse - liquide ionique contenant un sel d'onium à tâche spécifique (I)

### - exemple E1 :

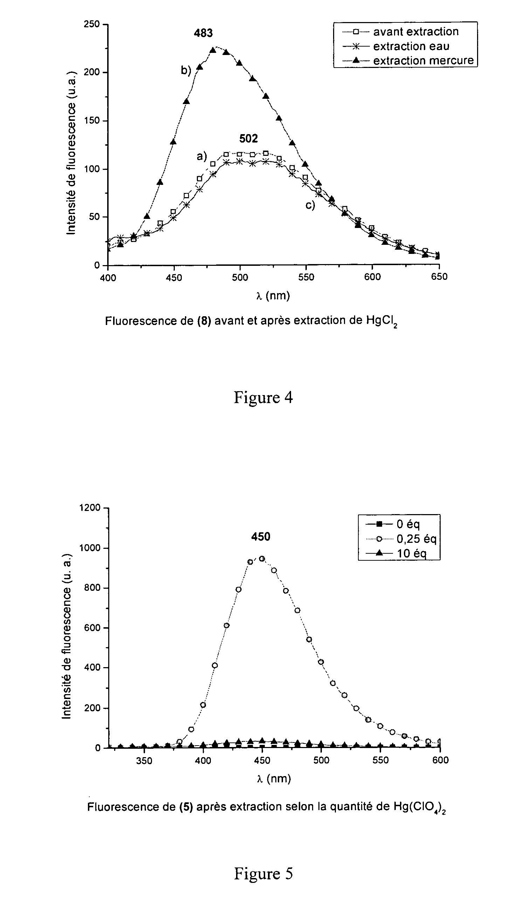

1 ml d'une solution aqueuse de HgCl₂ de concentration connue (10⁻²M) est ajoutée à 1ml d'une solution de **(8)** (5.0 x 10⁻⁴Mdans un liquide ionique (butylméthylpyrrolidinium bis(trifluoromethane sulfonamidure) [BMP][NTf₂]). Le système biphasique est agité pendant 5 minutes à l'aide d'un vortex afin d'atteindre l'équilibre. Après extraction, on laisse décanter les deux phases puis la fluorescence de la phase constituée de liquide ionique est mesurée entre 370 nm et 650 nm. Un signal de fluorescence est émis à 483nm. Comme illustré sur la figure 4. Sur cette figure on observe la fluorescence de **(8)** dans [BMP][NTf₂] avant extraction a), après extraction avec une solution aqueuse de HgCl₂ b), témoin avec de l'eau distillée c), λ_{exc} = 380 nm.

### - exemple E2 :

1 ml d'une solution aqueuse de Hg(ClO₄)₂ de concentration connue est ajoutée à 1 ml d'une solution de **(5)** (1.0 x 10⁻⁴M) dans un liquide ionique (butylméthylpyrrolidinium bis(trifluoromethane sulfonamidure) [BMP][NTf₂]). Le système biphasique est agité pendant 2 minutes à l'aide d'un vortex afin d'atteindre l'équilibre. Après extraction, on sépare les deux phases par centrifugation (2 min, 2000 tr/min) puis la fluorescence de la phase constituée de liquide ionique est mesurée entre 320 nm et 600 nm. Un signal de fluorescence est émis à 450 nm. Comme illustré sur la figure 5. Sur cette figure on observe la fluorescence de **(5)** dans [BMP][NTf₂] après extraction selon la quantité de Hg(ClO₄)₂ contenue dans la phase aqueuse, λ_{exc} = 313 nm.
- exemple E3 : Extraction liquide/liquide en microsystème et détection par fluorescence : exemple d'une partie d'un "kit microfluidique".

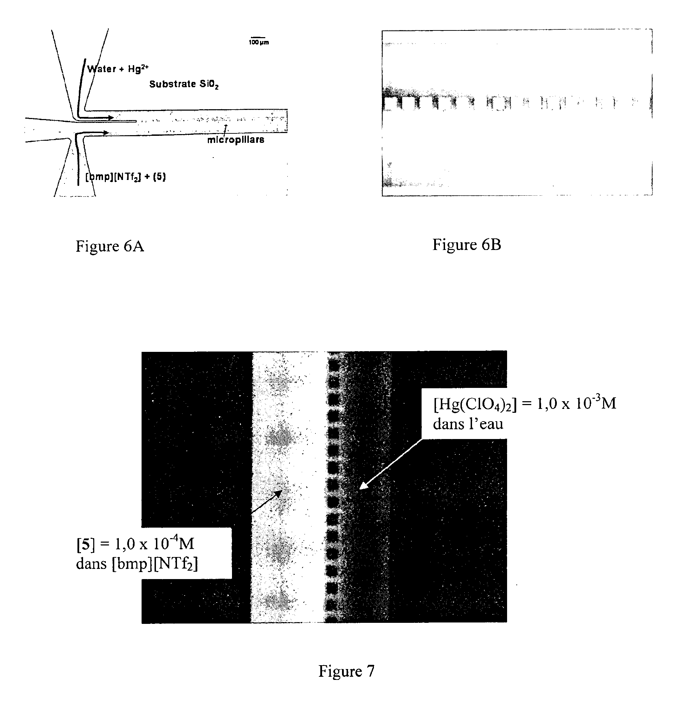

Dans le dispositif représenté sur les figures 6A et 6B, la puce consiste en un système microfluidique multi-canaux gravé dans le silicone et en un couvercle en Pyrex permettant de créer une cavité. L'interface bi phasique est stabilisée par des micro piliers carrés verticaux et les échanges d'espèces moléculaires est assuré par une série d'interfaces. La puce est composée de deux canaux de 5 mm de long, de 150 µm de profondeur et de 108 et 150 µm de largeur respectivement pour l'eau et pour le liquide ionique. Les micro piliers sont de 28x28 µm et espacés de 9,4 µm.

Protocole d'extraction : La solution aqueuse contenant les ions mercure Hg(ClO4)2 a été introduite à un flux choisi (entre 4 et 5 µl/min) dans le canal de largeur 108 µm. Ensuite la solution de (5) dans [bmp][NTf₂] a été introduite à un flux choisi (0,02 µL/min) dans le canal de largeur 150 µm. A la fin du remplissage, on a commencé la détection optique à l'aide d'un microscope d'épifluorescence. Les deux flux ont assuré la stabilité de l'interface et ont été maintenus pendant toute la durée de l'expérience. Les images de fluorescence (figure 7) sont prises toutes les dix minutes de façon à suivre l'évolution de la fluorescence dans la phase ionique liquide.

## Revendications

1. Molécule de formule (I) : 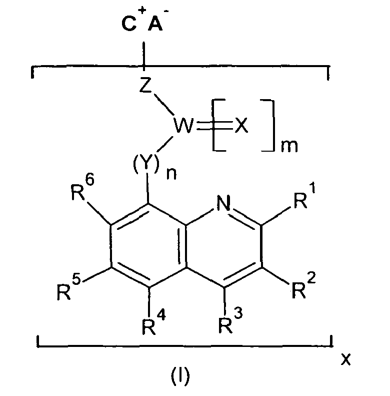 dans laquelle
X représente un groupement choisi parmi : O, NR, S, PR, Se ; et R représente un groupement choisi parmi : H, un groupement alkyle en C₁-C₁₂ comportant éventuellement un ou plusieurs hétéroatomes, un groupement alcényle en C₂-C₁₂ comportant éventuellement un ou plusieurs hétéroatomes, un groupement aryle en C₆-C₁₂ comportant éventuellement un ou plusieurs hétéroatomes, un groupement aralkyle en C₆-C₁₂ comportant éventuellement un ou plusieurs hétéroatomes,
Y représente un groupement choisi parmi : O, S, NR', PR', CR^{'}R^{''}, un groupement alcane di-yle en C₁-C₁₂ comportant éventuellement un ou plusieurs hétéroatomes, un groupement alcène di-yle en C₂-C₁₂ comportant éventuellement un ou plusieurs hétéroatomes, un groupement aryle en C₆-C₁₂ comportant éventuellement un ou plusieurs hétéroatomes, un groupement aralcane di-yle en C₆-C₁₂ comportant éventuellement un ou plusieurs hétéroatomes ; et R', R^{''}, identiques ou différents, représentent un groupement choisi parmi : H, un groupement alkyle en C₁-C₁₂ comportant éventuellement un ou plusieurs hétéroatomes, un groupement alcényle en C₂-C₁₂ comportant éventuellement un ou plusieurs hétéroatomes,un groupement aralkyle en C₆-C₁₂ comportant éventuellement un ou plusieurs hétéroatomes, un groupement aryle en C₆-C₁₂ comportant éventuellement un ou plusieurs hétéroatomes,
n est un entier choisi parmi : 0, 1,
m est un entier choisi parmi : 1, 2 ;
x est un entier choisi parmi : 1 et 2 ;
W représente un atome choisi parmi : C et S;
Z représente un groupement choisi parmi : un groupement alcane di-yle en C₁-C₁₂ comportant éventuellement un ou plusieurs hétéroéléments, un groupement alcène di-yle en C₂-C₁₂ comportant éventuellement un ou plusieurs hétéroéléments, un groupement aralcane di-yle en C₆-C₂₀ comportant éventuellement un ou plusieurs hétéroéléments, un groupement aryle en C₆-C₁₂ comportant éventuellement un ou plusieurs hétéroéléments, le ou les hétéroéléments étant choisis parmi : Cl, Br, O, S, NR''', PR''' ; et R"' représente un groupement choisi parmi : H, un groupement alkyle en C₁-C₁₂, un groupement alcényle en C₂-C₁₂, un groupement aryle en C₆-C₁₂, un groupement aralkyle en C₆-C₁₂ ;
R¹, R², R³, R⁴, R⁵, R⁶, identiques ou différents, représentent chacun un groupement choisi parmi la liste suivante : H, Cl, Br, un groupement alkyle en C₁-C₁₂ comportant éventuellement un ou plusieurs hétéroéléments, un groupement alcényle en C₂-C₁₂ comportant éventuellement un ou plusieurs hétéroéléments, un groupement aryle en C₆-C₁₂ comportant éventuellement un ou plusieurs hétéroéléments, un groupement aralkyle en C₆-C₁₂ comportant éventuellement un ou plusieurs hétéroéléments, OH, un groupement OR⁷, NH₂, un groupement NR⁷R⁸, SH, un groupement SR⁷ ; et R⁷, R⁸ sont choisis indépendamment l'un de l'autre parmi la liste suivante : un groupement alkyle en C₁-C₁₂, un groupement alcényle en C₂-C₁₂, un groupement aryle en C₆-C₁₂, un groupement aralkyle en C₆-C_{12 ;} le ou les hétéroéléments étant choisis parmi : Cl, Br, O, S, NR''', PR''' ; et R''' représente un groupement choisi parmi : H, un groupement alkyle en C₁-C₁₂, un groupement alcényle en C₂-C₁₂, un groupement aryle en C₆-C₁₂, un groupement aralkyle en C₆-C₁₂.
→ **C**⁺**A**⁻ représente un sel d'onium à tâche spécifique,
lorsque x=1, **C**⁺**A**⁻ peut être lié à Z soit par l'anion A⁻ soit par le cation C⁺,
lorsque x=2, l'anion **A**⁻ et le cation **C**⁺ sont liés chacun à un groupement Z, les deux groupements 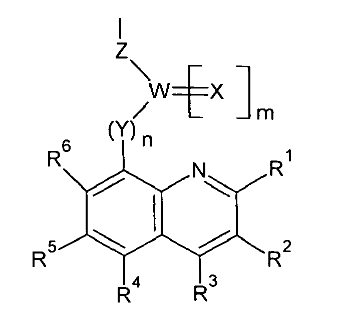 étant identiques ou différents, et **A**⁻ représente un groupement choisi parmi : hexafluorophosphate (PF₆⁻), bis(trifluorométhane sulfonamidure) (NTf₂⁻), tris (pentafluoroéthyl)trifluorophosphate (FAP), triflate (TfO⁻), nonaflate (NfO⁻).

2. Molécule selon la revendication 1, dans laquelle **C**⁺**A**⁻ représente un composé choisi parmi les sels d'ammonium, de phosphonium, de sulfonium ainsi que les sels résultant de la quaternarisation d'une amine, d'une phosphine, d'un thioéther ou d'un hétérocycle contenant un ou plusieurs héteroatomes tels que S, N, P.

3. Molécule selon la revendication 1 ou la revendication 2, dans laquelle **C⁺** est choisi parmi les ions ammonium, pyrrolidinium, phosphonium, imidazolium, pyridinium, pipéridinium, guanidinium.

4. Molécule selon l'une quelconque des revendications 1 à 3 dans laquelle l'une ou plusieurs des conditions suivantes sont vérifiées :
X représente un groupement choisi parmi : O, S,
Y représente un groupement choisi parmi : O, S, NH, un groupement alcane di-yle en C₁-C₆,
n = 1,
m = 1,
x=1,
W représente un atome de carbone : C,
Z représente un groupement choisi parmi : un groupement alcane di-yle en C₁-C₆ comportant éventuellement un ou plusieurs hétéroéléments, aralcane di-yle en C₆-C₂₀ comportant éventuellement un ou plusieurs hétéroéléments, le ou les hétéroéléments étant choisis parmi : O, S, NH,
R¹, R², R³, R⁴, R⁵, R⁶, identiques ou différents, représentent chacun un groupement choisi parmi la liste suivante : H, un groupement alkyle en C₁-C₆,
C⁺ représente un groupement

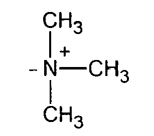

5. Molécule selon l'une quelconque des revendications 1 à 4 qui appartient à la liste suivante:

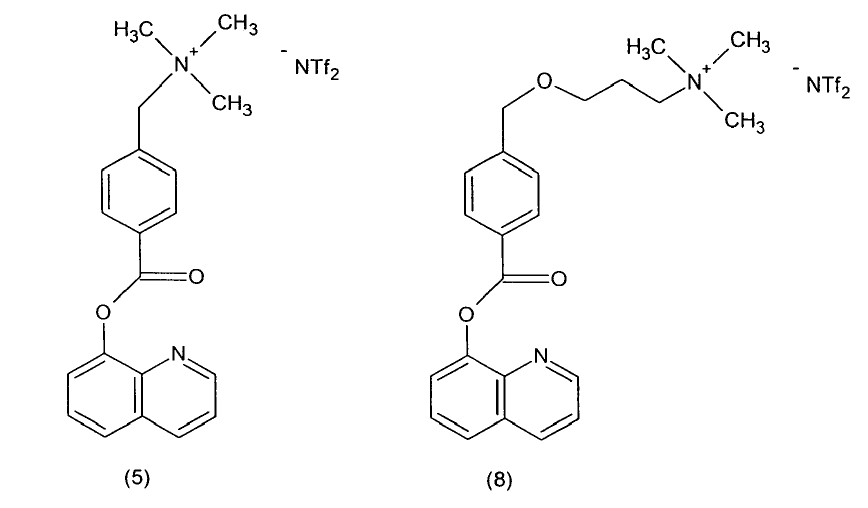

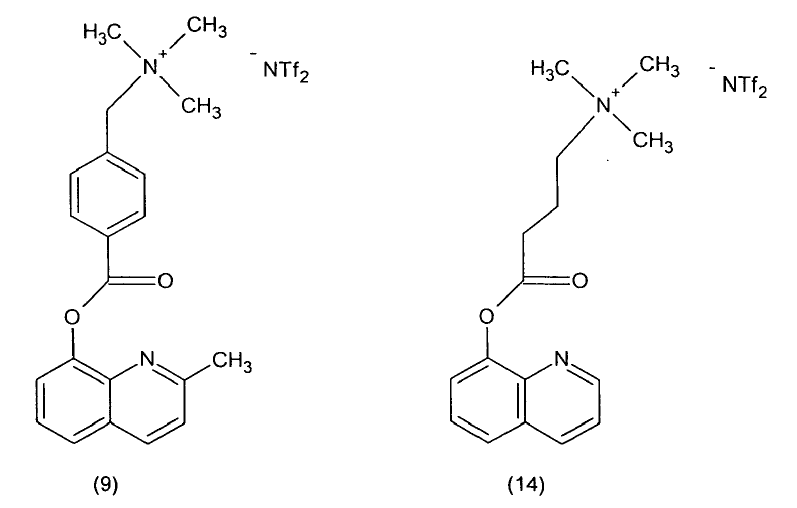

6. Composition comprenant au moins une molécule de formule (I) : 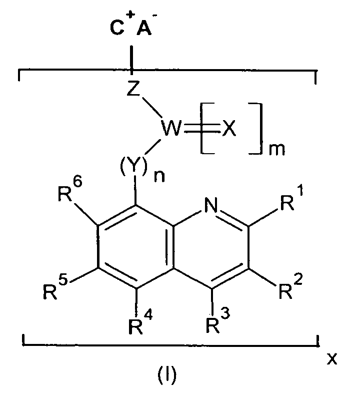 dans laquelle
X représente un groupement choisi parmi : O, NR, S, PR, Se ; et R représente un groupement choisi parmi : H, un groupement alkyle en C₁-C₁₂ comportant éventuellement un ou plusieurs hétéroatomes, un groupement alcényle en C₂-C₁₂ comportant éventuellement un ou plusieurs hétéroatomes, un groupement aryle en C₆-C₁₂ comportant éventuellement un ou plusieurs hétéroatomes, un groupement aralkyle en C₆-C₁₂ comportant éventuellement un ou plusieurs hétéroatomes,
Y représente un groupement choisi parmi : O, S, NR', PR', CR^{'}R^{"}, un groupement alcane di-yle en C₁-C₁₂ comportant éventuellement un ou plusieurs hétéroatomes, un groupement alcène di-yle en C₂-C₁₂ comportant éventuellement un ou plusieurs hétéroatomes, un groupement aryle en C₆-C₁₂ comportant éventuellement un ou plusieurs hétéroatomes, un groupement aralcane di-yle en C₆-C₁₂ comportant éventuellement un ou plusieurs hétéroatomes ; et R', R", identiques ou différents, représentent un groupement choisi parmi : H, un groupement alkyle en C₁-C₁₂ comportant éventuellement un ou plusieurs hétéroatomes, un groupement alcényle en C₂-C₁₂ comportant éventuellement un ou plusieurs hétéroatomes,un groupement aralkyle en C₆-C₁₂ comportant éventuellement un ou plusieurs hétéroatomes, un groupement aryle en C₆-C₁₂ comportant éventuellement un ou plusieurs hétéroatomes,
n est un entier choisi parmi : 0, 1,
m est un entier choisi parmi : 1, 2 ;
x est un entier choisi parmi : 1 et 2 ;
W représente un atome choisi parmi : C et S;
Z représente un groupement choisi parmi : un groupement alcane di-yle en C₁-C₁₂ comportant éventuellement un ou plusieurs hétéroéléments, un groupement alcène di-yle en C₂-C₁₂ comportant éventuellement un ou plusieurs hétéroéléments, un groupement aralcane di-yle en C₆-C₂₀ comportant éventuellement un ou plusieurs hétéroéléments, un groupement aryle en C₆-C₁₂ comportant éventuellement un ou plusieurs hétéroéléments, le ou les hétéroéléments étant choisis parmi : Cl, Br, O, S, NR''', PR"' ; et R"' représente un groupement choisi parmi : H, un groupement alkyle en C₁-C₁₂, un groupement alcényle en C₂-C₁₂, un groupement aryle en C₆-C₁₂, un groupement aralkyle en C₆-C₁₂ ;
R¹, R², R³, R⁴, R⁵, R⁶, identiques ou différents, représentent chacun un groupement choisi parmi la liste suivante : H, Cl, Br, un groupement alkyle en C₁-C₁₂ comportant éventuellement un ou plusieurs hétéroéléments, un groupement alcényle en C₂-C₁₂ comportant éventuellement un ou plusieurs hétéroéléments, un groupement aryle en C₆-C₁₂ comportant éventuellement un ou plusieurs hétéroéléments, un groupement aralkyle en C₆-C₁₂ comportant éventuellement un ou plusieurs hétéroéléments, OH, un groupement OR⁷, NH₂, un groupement NR⁷R⁸, SH, un groupement SR⁷; et R⁷, R⁸ sont choisis indépendamment l'un de l'autre parmi la liste suivante : un groupement alkyle en C₁-C₁₂, un groupement alcényle en C₂-C₁₂, un groupement aryle en C₆-C₁₂, un groupement aralkyle en C₆-C_{12 ;} le ou les hétéroéléments étant choisis parmi : Cl, Br, O, S, NR''', PR''' ; et R''' représente un groupement choisi parmi : H, un groupement alkyle en C₁-C₁₂, un groupement alcényle en C₂-C₁₂, un groupement aryle en C₆-C₁₂, un groupement aralkyle en C₆-C₁₂.
→ **C⁺A⁻** représente un sel d'onium à tâche spécifique,
lorsque x=1, **C⁺A⁻** peut être lié à Z soit par l'anion A⁻ soit par le cation C⁺,
lorsque x=2, l'anion **A⁻** et le cation **C⁺** sont liés chacun à un groupement Z, les deux groupements 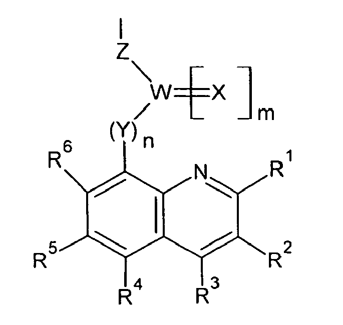 étant identiques ou différents et au moins un solvant, choisi parmi les matrices liquides ioniques à température ambiante hydrophobe.

7. Composition selon la revendication 6, dans laquelle au moins un solvant est choisi parmi : les N,N-dialkylpyrrolidinium, les N,N-dialkylpipéridinium, les N,N trialkylammonium.

8. Procédé d'extraction de métaux dans une composition aqueuse, ce procédé comportant au moins une étape de mise en contact de la composition aqueuse dans laquelle sont présents le ou les métaux, avec un composé de formule (I) : 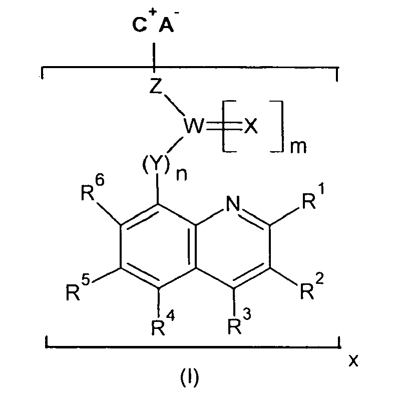 dans laquelle
X représente un groupement choisi parmi : O, NR, S, PR, Se ; et R représente un groupement choisi parmi : H, un groupement alkyle en C₁-C₁₂ comportant éventuellement un ou plusieurs hétéroatomes, un groupement alcényle en C₂-C₁₂ comportant éventuellement un ou plusieurs hétéroatomes, un groupement aryle en C₆-C₁₂ comportant éventuellement un ou plusieurs hétéroatomes, un groupement aralkyle en C₆-C₁₂ comportant éventuellement un ou plusieurs hétéroatomes,
Y représente un groupement choisi parmi : O, S, NR', PR', CR^{'}R^{"}, un groupement alcane di-yle en C₁-C₁₂ comportant éventuellement un ou plusieurs hétéroatomes, un groupement alcène di-yle en C₂-C₁₂ comportant éventuellement un ou plusieurs hétéroatomes, un groupement aryle en C₆-C₁₂ comportant éventuellement un ou plusieurs hétéroatomes, un groupement aralcane di-yle en C₆-C₁₂ comportant éventuellement un ou plusieurs hétéroatomes ; et R', R^{''}, identiques ou différents, représentent un groupement choisi parmi : H, un groupement alkyle en C₁-C₁₂ comportant éventuellement un ou plusieurs hétéroatomes, un groupement alcényle en C₂-C₁₂ comportant éventuellement un ou plusieurs hétéroatomes,un groupement aralkyle en C₆-C₁₂ comportant éventuellement un ou plusieurs hétéroatomes, un groupement aryle en C₆-C₁₂ comportant éventuellement un ou plusieurs hétéroatomes,
n est un entier choisi parmi : 0, 1,
m est un entier choisi parmi : 1, 2 ;
x est un entier choisi parmi : 1 et 2 ;
W représente un atome choisi parmi : C et S;
Z représente un groupement choisi parmi : un groupement alcane di-yle en C₁-C₁₂ comportant éventuellement un ou plusieurs hétéroéléments, un groupement alcène di-yle en C₂-C₁₂ comportant éventuellement un ou plusieurs hétéroéléments, un groupement aralcane di-yle en C₆-C₂₀ comportant éventuellement un ou plusieurs hétéroéléments, un groupement aryle en C₆-C₁₂ comportant éventuellement un ou plusieurs hétéroéléments, le ou les hétéroéléments étant choisis parmi : Cl, Br, O, S, NR''', PR''' ; et R"' représente un groupement choisi parmi : H, un groupement alkyle en C₁-C₁₂, un groupement alcényle en C₂-C₁₂, un groupement aryle en C₆-C₁₂, un groupement aralkyle en C₆-C₁₂ ;
R¹, R², R³, R⁴, R⁵, R⁶, identiques ou différents, représentent chacun un groupement choisi parmi la liste suivante : H, Cl, Br, un groupement alkyle en C₁-C₁₂ comportant éventuellement un ou plusieurs hétéroéléments, un groupement alcényle en C₂-C₁₂ comportant éventuellement un ou plusieurs hétéroéléments, un groupement aryle en C₆-C₁₂ comportant éventuellement un ou plusieurs hétéroéléments, un groupement aralkyle en C₆-C₁₂ comportant éventuellement un ou plusieurs hétéroéléments, OH, un groupement OR⁷, NH₂, un groupement NR⁷R⁸, SH, un groupement SR⁷; et R⁷, R⁸ sont choisis indépendamment l'un de l'autre parmi la liste suivante : un groupement alkyle en C₁-C₁₂, un groupement alcényle en C₂-C₁₂, un groupement aryle en C₆-C₁₂, un groupement aralkyle en C₆-C_{12;} le ou les hétéroéléments étant choisis parmi : Cl, Br, O, S, NR''', PR''' ; et R''' représente un groupement choisi parmi : H, un groupement alkyle en C₁-C₁₂, un groupement alcényle en C₂-C₁₂, un groupement aryle en C₆-C₁₂, un groupement aralkyle en C₆-C₁₂.
→ **C**⁺**A**⁻ représente un sel d'onium à tâche spécifique,
lorsque x=1, **C**⁺**A**⁻ peut être lié à Z soit par l'anion A⁻ soit par le cation C⁺,
lorsque x=2, l'anion **A**⁻ et le cation **C**⁺ sont liés chacun à un groupement Z, les deux groupements 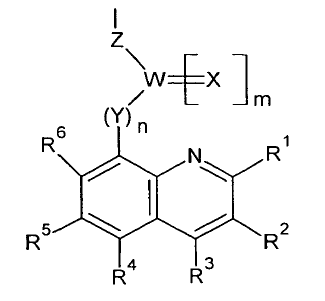 étant identiques ou différents.

9. Procédé selon la revendication 8, qui est mis en oeuvre au sein d'un dispositif microfluidique.

10. Procédé selon l'une quelconque des revendications 8 et 9, dans lequel **A**⁻ représente un groupement choisi parmi : hexafluorophosphate (PF₆⁻), bis(trifluorométhane sulfonamidure) (NTf₂⁻), tris (pentafluoroéthyl)trifluorophosphate (FAP), triflate (TfO⁻), nonaflate (NfO⁻).

11. Procédé selon l'une quelconque des revendications 8 à 10, dans lequel **C**⁺**A**⁻ représente un composé choisi parmi les sels d'ammonium, de phosphonium, de sulfonium ainsi que les sels résultant de la quaternarisation d'une amine, d'une phosphine, d'un thioéther ou d'un hétérocycle contenant un ou plusieurs héteroatomes tels que S, N, P.

12. Procédé selon l'une quelconque des revendications 8 à 11, dans lequel **C**⁺ est choisi parmi les ions ammonium, pyrrolidinium, phosphonium, imidazolium, pyridinium, pipéridinium, guanidinium.

13. Procédé de détection de métaux dans une composition aqueuse, ce procédé comportant au moins une étape selon l'une des revendications 8 à 12 et au moins une étape de mesure de fluorescence.

14. Kit pour la détection de métaux en milieu aqueux comprenant un dispositif microfluidique et au moins un composé de formule (I) tel que défini dans l'une quelconque des revendication 8 à 12.

15. Kit pour la détection de métaux en milieu aqueux comprenant au moins un composé de formule (I) tel que défini dans l'une quelconque des revendication 8 à 12 et un détecteur de fluorescence.
